# EUROPEAN PATENT APPLICATION

(11) **EP 4 174 087 A1**
(43) Date of publication of application: **03.05.2023**
(21) Application number: 21833511.5
(22) Date of filing: 30.06.2021
(51) Int. Cl.: C07K 16/28, C12N 15/13, A61K 39/395, A61P 37/02

(54) **DRUG FOR TREATING TUMOR**

(30) Priority: 30.06.2020 CN 202010619083; 30.06.2020 CN 202010619111; 30.06.2020 CN 202010620778
(71) Applicant: CHIA TAI TIANQING PHARMACEUTICAL GROUP CO., LTD., Lianyungang, Jiangsu 222062 (CN)
(72) Inventor: SU, Nan, Nanjing, Jiangsu 211100 (CN); ZHANG, Xiquan, Nanjing, Jiangsu 211100 (CN); CHEN, Jie, Nanjing, Jiangsu 211100 (CN); WANG, Xunqiang, Nanjing, Jiangsu 211100 (CN); LI, Kun, Nanjing, Jiangsu 211100 (CN); YU, Ding, Nanjing, Jiangsu 211100 (CN); WANG, Rongliang, Nanjing, Jiangsu 211100 (CN)
(74) Representative: v. Bezold & Partner Patentanwälte - PartG mbB
(86) International application number: PCT/CN2021/103687
(87) International publication number: WO 2022/002153

(57) **Abstract**

The present invention belongs to the field of biomedicines, relates to a drug for treating tumors, and provides use of an anti-PD-L1 antibody or a pharmaceutical composition thereof in preparation of a drug for treating endometrial cancer. Also provided is use of the anti-PD-L1 antibody or the pharmaceutical composition thereof in preparation of a drug for treating a MSI-H and/or dMMR tumor. In addition, also provided is use of a pharmaceutical combination of the anti-PD-L1 antibody and Anlotinib or a pharmaceutically acceptable salt thereof, or a pharmaceutical composition thereof in preparation of a drug for treating endometrial cancer.

## Description

### TECHNICAL FIELD

The present application belongs to the field of biomedicine, and particularly relates to a medicament for treating a tumor.

### BACKGROUND

Endometrial cancer (EC) is a group of epithelial malignant tumors occurring in the endometrium, also known as corpus uteri cancer, and is one of the three most common malignant tumors of the female reproductive tract. It is most common in perimenopausal and postmenopausal women. With the increase in the average life span of the population and the change in lifestyle, the incidence of endometrial cancer has been on a continuously rising trend for the last 20 years and patients have become increasingly younger. In western countries, the incidence of endometrial cancer has ranked first among malignant tumors of the female reproductive system, while in China, endometrial cancer is the second most common gynecological malignant tumor after cervical cancer and accounts for about 20%-30% of gynecological malignant tumors. The incidence of endometrial cancer in some developed cities has ranked first in gynecological malignant tumors. The postoperative recurrence rate of patients with stage I and stage II disease is about 15%, with 50%-70% of the recurrences being symptomatic. Most recurrences occur within 3 years after treatment. Treatment for recurrent tumors confined to vagina or pelvis can still achieve good effect. Patients with an isolated vaginal recurrence have a 5-year survival rate of 50%-70% after radiotherapy.

In principle, the treatment of endometrial cancer is based on surgery, supplemented by multimodality therapy such as radiotherapy, drug therapy, and hormone. For estrogen-dependent and nulliparous patients with early-stage endometrial cancer, conservative treatment with a progestogen is often used to preserve fertility; for patients with advanced-stage endometrial cancer, appropriate chemotherapy is adopted to reduce the tumor volume before operation, and reasonable selection of chemotherapy drugs can significantly improve the surgical resection rate; for metastatic endometrial cancer and endometrial cancer that recurs after surgery, hormone drugs such as progestogen, gonadotropin-releasing hormone analogue and aromatase inhibitor, and therapeutic drugs such as paclitaxel, carboplatin and doxorubicin are commonly used. With the emergence of chemotherapy resistance, targeted drug therapy such as pembrolizumab, avelumab, bevacizumab, sorafenib and sunitinib has important significance for improving the efficacy and prognosis of endometrial cancer.

Microsatellite (MS) refers to a DNA sequence in which a few nucleotides (mostly 1-6) are tandemly repeated in units in the cell genome, and is also called short tandem repeat (STR). When the function of DNA mismatch repair (MMR) is abnormal, the copy errors of the microsatellite cannot be corrected and are accumulated continuously, resulting in changes in the length or base composition of microsatellite, which is called microsatellite instability (MSI), and at the same time leading to a hypermutated phenotype in the genome. MSI can be classified into 3 categories according to degree: microsatellite instability-high (MSI-H), microsatellite instability-low (MSI-L), and microsatellite stability (MSS).

Programmed death factor-1 and its ligands (PD-1/PD-L1) are a pair of immune co-stimulatory factors. PD-1 normally exerts an immunomodulatory effect through its ligand, PD-L1. Activation of the PD-1/PD-L1 signaling pathway may lead to formation of an immunosuppressive tumor microenvironment, so that tumor cells can escape from immune monitoring and killing of organisms, while blocking the PD-1/PD-L1 signaling pathway may reverse the tumor immune microenvironment and enhance endogenous anti-tumor immune effects.

### BRIEF SUMMARY

In one aspect, the present application provides use of an anti-PD-L1 antibody in preparing a medicament for treating MSI-H endometrial cancer and/or dMMR endometrial cancer.

In another aspect, the present application provides use of a pharmaceutical composition comprising an anti-PD-L1 antibody in preparing a medicament for treating MSI-H endometrial cancer and/or dMMR endometrial cancer.

In another aspect, the present application further provides use of an anti-PD-L1 antibody in treating MSI-H endometrial cancer and/or dMMR endometrial cancer.

In another aspect, the present application further provides use of a pharmaceutical composition comprising an anti-PD-L1 antibody in treating MSI-H endometrial cancer and/or dMMR endometrial cancer.

In some embodiments, the pharmaceutical composition further comprises a pharmaceutically acceptable carrier.

In one aspect, the present application provides a therapeutic combination for use in treating endometrial cancer, which comprises: an anti-PD-L1 antibody, and anlotinib or a pharmaceutically acceptable salt thereof. In some embodiments, provided is a therapeutic combination for use in treating non-MSI-H endometrial cancer and/or non-dMMR endometrial cancer, which comprises: an anti-PD-L1 antibody, and anlotinib or a pharmaceutically acceptable salt thereof.

In another aspect, the present application provides use of a therapeutic combination of an anti-PD-L1 antibody, and anlotinib or a pharmaceutically acceptable salt thereof in preparing a medicament for treating endometrial cancer. In some embodiments, provided is use of a therapeutic combination comprising an anti-PD-L1 antibody, and anlotinib or a pharmaceutically acceptable salt thereof in preparing a medicament for treating endometrial cancer. In some embodiments, provided is use of a therapeutic combination of an anti-PD-L1 antibody, and anlotinib or a pharmaceutically acceptable salt thereof in preparing a medicament for treating non-MSI-H endometrial cancer and/or non-dMMR endometrial cancer. In some embodiments, provided is use of a therapeutic combination comprising an anti-PD-L1 antibody, and anlotinib or a pharmaceutically acceptable salt thereof in preparing a medicament for treating non-MSI-H endometrial cancer and/or non-dMMR endometrial cancer.

In another aspect, the present application provides use of a therapeutic combination of an anti-PD-L1 antibody, and anlotinib or a pharmaceutically acceptable salt thereof in treating endometrial cancer. In some embodiments, provided is use of a therapeutic combination comprising an anti-PD-L1 antibody, and anlotinib or a pharmaceutically acceptable salt thereof in treating endometrial cancer. In some embodiments, the present application provides use of a therapeutic combination of an anti-PD-L1 antibody, and anlotinib or a pharmaceutically acceptable salt thereof in treating non-MSI-H endometrial cancer and/or non-dMMR endometrial cancer. In some embodiments, the present application provides use of a therapeutic combination comprising an anti-PD-L1 antibody, and anlotinib or a pharmaceutically acceptable salt thereof in treating non-MSI-H endometrial cancer and/or non-dMMR endometrial cancer.

In some embodiments, the therapeutic combination further comprises a pharmaceutically acceptable carrier.

In one aspect, the present application provides use of an anti-PD-L1 antibody in preparing a medicament for treating MSI-H and/or dMMR tumors. In some embodiments, provided is use of an anti-PD-L1 antibody in preparing a medicament for treating MSI-H and/or dMMR malignant tumors. In some embodiments, provided is use of an anti-PD-L1 antibody in preparing a medicament for treating MSI-H and/or dMMR solid tumors. In some embodiments, provided is use of an anti-PD-L1 antibody in preparing a medicament for treating MSI-H and/or dMMR malignant solid tumors.

In another aspect, the present application provides use of a pharmaceutical composition comprising an anti-PD-L1 antibody in preparing a medicament for treating MSI-H and/or dMMR tumors. In some embodiments, provided is use of a pharmaceutical composition comprising an anti-PD-L1 antibody in preparing a medicament for treating MSI-H and/or dMMR malignant tumors. In some embodiments, provided is use of a pharmaceutical composition comprising an anti-PD-L1 antibody in preparing a medicament for treating MSI-H and/or dMMR solid tumors. In some embodiments, provided is use of a pharmaceutical composition comprising an anti-PD-L1 antibody in preparing a medicament for treating MSI-H and/or dMMR malignant solid tumors.

In another aspect, the present application further provides use of an anti-PD-L1 antibody for treating MSI-H and/or dMMR tumors. In some embodiments, provided is use of an anti-PD-L1 antibody in treating MSI-H and/or dMMR malignant tumors. In some embodiments, provided is use of an anti-PD-L1 antibody in treating MSI-H and/or dMMR solid tumors. In some embodiments, provided is use of an anti-PD-L1 antibody in treating MSI-H and/or dMMR malignant solid tumors.

In another aspect, the present application further provides use of a pharmaceutical composition comprising an anti-PD-L1 antibody in treating MSI-H and/or dMMR tumors. In some embodiments, provided is use of a pharmaceutical composition comprising an anti-PD-L1 antibody in treating MSI-H and/or dMMR malignant tumors. In some embodiments, provided is use of a pharmaceutical composition comprising an anti-PD-L1 antibody in treating MSI-H and/or dMMR solid tumors. In some embodiments, provided is use of a pharmaceutical composition comprising an anti-PD-L1 antibody in treating MSI-H and/or dMMR malignant solid tumors.

In yet another aspect, the present application further provides a method for treating MSI-H endometrial cancer and/or dMMR endometrial cancer, which comprises administering to a patient in need thereof a therapeutically effective amount of an anti-PD-L1 antibody.

In yet another aspect, the present application provides a method for treating MSI-H endometrial cancer and/or dMMR endometrial cancer, which comprises administering to a patient in need thereof a therapeutically effective amount of a pharmaceutical composition comprising an anti-PD-L1 antibody.

In yet another aspect, the present application further provides a method for treating endometrial cancer, which comprises administering to a patient in need thereof a therapeutically effective amount of an anti-PD-L1 antibody, and anlotinib or a pharmaceutically acceptable salt thereof. In some embodiments, the present application further provides a method for treating non-MSI-H endometrial cancer and/or non-dMMR endometrial cancer, which comprises administering to a patient in need thereof a therapeutically effective amount of an anti-PD-L1 antibody, and anlotinib or a pharmaceutically acceptable salt thereof. In some embodiments, the anti-PD-L1 antibody and anlotinib or the pharmaceutically acceptable salt thereof can be administered simultaneously, nonsimultaneously, or sequentially. In some embodiments, the present application further provides a method for treating endometrial cancer, which comprises administering to a patient in need thereof a therapeutically effective amount of a pharmaceutical composition comprising an anti-PD-L1 antibody, and anlotinib or a pharmaceutically acceptable salt thereof.

In some embodiments, the present application further provides a method for treating non-MSI-H endometrial cancer and/or non-dMMR endometrial cancer, which comprises administering to a patient in need thereof a therapeutically effective amount of a pharmaceutical composition of an anti-PD-L1 antibody, and a pharmaceutical composition of anlotinib or a pharmaceutically acceptable salt thereof. In some embodiments, the pharmaceutical composition of the anti-PD-L1 antibody, and the pharmaceutical composition of anlotinib or the pharmaceutically acceptable salt thereof, can be administered simultaneously, nonsimultaneously, or sequentially. In some embodiments, the present application further provides a method for treating non-MSI-H endometrial cancer and/or non-dMMR endometrial cancer, which comprises administering to a patient in need thereof a therapeutically effective amount of a pharmaceutical composition comprising an anti-PD-L1 antibody, and anlotinib or a pharmaceutically acceptable salt thereof.

In yet another aspect, the present application further provides a method for treating MSI-H and/or dMMR tumors, which comprises administering to a patient in need thereof a therapeutically effective amount of an anti-PD-L1 antibody. In some embodiments, provided is a method for treating MSI-H and/or dMMR malignant tumors, which comprises administering to a patient in need thereof a therapeutically effective amount of an anti-PD-L1 antibody. In some embodiments, provided is a method for treating MSI-H and/or dMMR malignant solid tumors, which comprises administering to a patient in need thereof a therapeutically effective amount of an anti-PD-L1 antibody. In yet another aspect, the present application further provides a method for treating MSI-H and/or dMMR tumors, which comprises administering to a patient in need thereof a therapeutically effective amount of a pharmaceutical composition comprising an anti-PD-L1 antibody. In some embodiments, provided is a method for treating MSI-H and/or dMMR malignant tumors, which comprises administering to a patient in need thereof a therapeutically effective amount of a pharmaceutical composition comprising an anti-PD-L1 antibody. In some embodiments, provided is a method for treating MSI-H and/or dMMR malignant solid tumors, which comprises administering to a patient in need thereof a therapeutically effective amount of a pharmaceutical composition comprising an anti-PD-L1 antibody.

In still another aspect, the present application provides a kit for use in treating MSI-H endometrial cancer and/or dMMR endometrial cancer, which comprises an anti-PD-L1 antibody. In some embodiments, the kit further comprises instructions for treating endometrial cancer.

In yet another aspect, the present application provides a kit for use in treating MSI-H endometrial cancer and/or dMMR endometrial cancer, which comprises a pharmaceutical composition comprising an anti-PD-L1 antibody; in some embodiments, the kit further comprises instructions for treating endometrial cancer.

In still another aspect, the present application provides a kit for use in treating endometrial cancer, which comprises: an anti-PD-L1 antibody, and anlotinib or a pharmaceutically acceptable salt thereof; in some embodiments, the anti-PD-L1 antibody is contained in a first compartment, and anlotinib or the pharmaceutically acceptable salt thereof is contained in a second compartment; they can be administered simultaneously, nonsimultaneously, or sequentially to a patient in need. In some embodiments, the kit further comprises instructions for combined use of an anti-PD-L1 antibody, and anlotinib or a pharmaceutically acceptable salt thereof in treating endometrial cancer. In some embodiments, the kit comprises: a pharmaceutical composition of the anti-PD-L1 antibody, and a pharmaceutical composition of anlotinib or the pharmaceutically acceptable salt thereof.

In some embodiments, the present application provides a kit for treating non-MSI-H endometrial cancer and/or non-dMMR endometrial cancer, comprising: an anti-PD-L1 antibody; and anlotinib or a pharmaceutically acceptable salt thereof, wherein: the anti-PD-L1 antibody is contained in a first compartment, and anlotinib or the pharmaceutically acceptable salt thereof is contained in a second compartment; they can be administered simultaneously, nonsimultaneously, or sequentially to a patient in need. The kit further comprises instructions for combined use of an anti-PD-L1 antibody, and anlotinib or a pharmaceutically acceptable salt thereof in treating non-MSI-H endometrial cancer and/or non-dMMR endometrial cancer. In some embodiments, the kit comprises: a pharmaceutical composition of the anti-PD-L1 antibody, and a pharmaceutical composition of anlotinib or the pharmaceutically acceptable salt thereof.

In still another aspect, the present application provides a kit for use in treating MSI-H and/or dMMR tumors, which comprises an anti-PD-L1 antibody; in some embodiments, the kit further comprises instructions for treating MSI-H and/or dMMR tumors. In some embodiments, the present application provides a kit for use in treating MSI-H and/or dMMR malignant tumors, which comprises an anti-PD-L1 antibody; in some embodiments, the kit further comprises instructions for treating MSI-H and/or dMMR malignant tumors. In some embodiments, the present application provides a kit for use in treating MSI-H and/or dMMR malignant solid tumors, which comprises an anti-PD-L1 antibody; in some embodiments, the kit further comprises instructions for treating MSI-H and/or dMMR malignant solid tumors.

In yet another aspect, the present application provides a kit for use in treating MSI-H and/or dMMR tumors, which comprises a pharmaceutical composition comprising an anti-PD-L1 antibody; in some embodiments, the kit further comprises instructions for treating MSI-H and/or dMMR tumors. In some embodiments, the present application provides a kit for use in treating MSI-H and/or dMMR malignant tumors, which comprises a pharmaceutical composition comprising an anti-PD-L1 antibody. In some embodiments, the kit further comprises instructions for treating MSI-H and/or dMMR malignant tumors. In some embodiments, the present application provides a kit for treating MSI-H and/or dMMR malignant solid tumors, which comprises a pharmaceutical composition comprising an anti-PD-L1 antibody. In some embodiments, the kit further comprises instructions for treating MSI-H and/or dMMR malignant solid tumors.

The amount and regimen of the anti-PD-L1 antibody administered may be determined according to the severity of the disease, the response of the disease, any treatment-related toxicity, and the age and health of a patient. For example, in some embodiments, the daily dose of the anti-PD-L1 antibody administered may be 600-2400 mg, and in some embodiments, the daily dose of the anti-PD-L1 antibody administered may be 600 mg, 800 mg, 1000 mg, 1200 mg, 1400 mg, 1600 mg, 1800 mg, 2000 mg, 2200 mg, or 2400 mg. In some embodiments, the anti-PD-L1 antibody is administered parenterally. In some embodiments, the anti-PD-L1 antibody is administered intravenously. In some embodiments, the anti-PD-L1 antibody is administered at a concentration of 10-60 mg/mL. In some embodiments, the anti-PD-L1 antibody is administered at a concentration of 10 mg/mL., 20 mg/mL., 30 mg/mL., 40 mg/mL., 50 mg/mL. or 60 mg/mL. In some embodiments, for the anti-PD-L1 antibody, one treatment cycle is every 1 week, every 2 weeks, every 3 weeks, or every 4 weeks. In some embodiments, the anti-PD-L1 antibody is administered once per treatment cycle. In some embodiments, the anti-PD-L1 antibody is administered once a week, every 2 weeks, every 3 weeks, or every 4 weeks. In some embodiments, for the anti-PD-L1 antibody, every 3 weeks are counted as one treatment cycle. In some embodiments, the anti-PD-L1 antibody is administered once every 3 weeks, or every 4 weeks.

In some embodiments, the anti-PD-L1 antibody is administered at a dose of 600-2400 mg per treatment cycle. In some embodiments, the anti-PD-L1 antibody is administered at a dose of 600 mg, 800 mg, 1000 mg, 1200 mg, 1400 mg, 1600 mg, 1800 mg, 2000 mg, 2200 mg, or 2400 mg per treatment cycle. In some embodiments, the anti-PD-L1 antibody is administered at a dose of 1200 mg per treatment cycle. In some embodiments, the anti-PD-L1 antibody is administered on the first day of each treatment cycle. In some specific embodiments, every 3 weeks are counted as one treatment cycle. In some specific embodiments, the anti-PD-L1 antibody is administered once every three weeks at a dose of 600-2400 mg. In some embodiments, the 3 weeks (21 days) are counted as one treatment cycle, and the anti-PD-L1 antibody is administered to the patient in need thereof on the first day of each treatment cycle. In some specific embodiments, the anti-PD-L1 antibody is administered once on the first day of every three weeks at a dose of 600-2400 mg. In some specific embodiments, the anti-PD-L1 antibody is administered once on the first day of every three weeks at a dose of 1200 mg.

In some embodiments, the therapeutic combination described herein comprises: an anti-PD-L1 humanized monoclonal antibody, and anlotinib or a pharmaceutically acceptable salt thereof.

In some embodiments, the therapeutic combination comprising an anti-PD-L1 antibody, and anlotinib or a pharmaceutically acceptable salt thereof described herein comprises: a pharmaceutical composition of the anti-PD-L1 antibody, and a pharmaceutical composition of anlotinib or the pharmaceutically acceptable salt thereof. In some embodiments, the pharmaceutical composition further comprises a pharmaceutically acceptable carrier.

In some embodiments, the therapeutic combination comprising an anti-PD-L1 antibody, and anlotinib or a pharmaceutically acceptable salt thereof described herein comprises: a pharmaceutical composition comprising 600-2400 mg of the anti-PD-L1 antibody. In some embodiments, the therapeutic combination comprises: a pharmaceutical composition comprising 600-2400 mg of an anti-PD-L1 antibody, wherein the pharmaceutical composition of the anti-PD-L1 antibody is in a unit dose or in multiple doses.

In some embodiments, the therapeutic combination comprising an anti-PD-L1 antibody, and anlotinib or a pharmaceutically acceptable salt thereof described herein comprises: a pharmaceutical composition of 6 mg to 12 mg of anlotinib or a pharmaceutically acceptable salt thereof. In some embodiments, the therapeutic combination comprises: a pharmaceutical composition of anlotinib or a pharmaceutically acceptable salt thereof at a unit dose of 6 mg, 8 mg, 10 mg and/or 12 mg.

In some embodiments, the therapeutic combination comprising an anti-PD-L1 antibody, and anlotinib or a pharmaceutically acceptable salt thereof described herein comprises: a pharmaceutical composition comprising 600-2400 mg of an anti-PD-L1 antibody, and a pharmaceutical composition of anlotinib or a pharmaceutically acceptable salt thereof at a unit dose of 6 mg, 8 mg, 10 mg and/or 12 mg, wherein the pharmaceutical composition of the anti-PD-L1 antibody is in a unit dose or in multiple doses. In some embodiments, the therapeutic combination comprises: a pharmaceutical composition comprising 600-2400 mg of an anti-PD-L1 antibody provided in multiple-dose form, and a pharmaceutical composition of anlotinib or a pharmaceutically acceptable salt thereof at a unit dose of 6 mg, 8 mg, 10 mg and/or 12 mg. In some embodiments, the therapeutic combination is a formulation suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib or a pharmaceutically acceptable salt thereof.

In some embodiments, a therapeutic combination comprising an anti-PD-L1 antibody, and anlotinib or a pharmaceutically acceptable salt thereof described herein comprises: the anti-PD-L1 antibody and anlotinib or the pharmaceutically acceptable salt thereof at a weight ratio of (0.35-29):1, (3.5-29):1, (3.5-14.5):1, or (7-14.5):1. The anti-PD-L1 antibody and anlotinib or the pharmaceutically acceptable salt thereof can be separately packaged or packaged together. Anlotinib can be packaged in multiple aliquots (e.g., 2 aliquots, 7 aliquots, 14 aliquots, 28 aliquots or more); the anti-PD-L1 antibody can be packaged in a single aliquot or in multiple aliquots (e.g., 2 aliquots, 4 aliquots or more).

In some embodiments, in the therapeutic combination comprising an anti-PD-L1 antibody, and anlotinib or a pharmaceutically acceptable salt thereof described herein, the anti-PD-L1 antibody and anlotinib or the pharmaceutically acceptable salt thereof are each in the form of a pharmaceutical composition and can be administered simultaneously, nonsimultaneously, or sequentially.

In some embodiments, the therapeutic combination of an anti-PD-L1 antibody, and anlotinib or a pharmaceutically acceptable salt thereof disclosed herein comprises a pharmaceutical composition of the anti-PD-L1 antibody and a pharmaceutical composition of anlotinib, wherein the anti-PD-L1 antibody is prepared in a unit dose or in multiple doses suitable for providing 600-2400 mg of the anti-PD-L1 antibody for a patient at first administration, and anlotinib or the pharmaceutically acceptable salt thereof is prepared in a unit dose suitable for providing 6 mg, 8 mg, 10 mg and/or 12 mg of anlotinib or the pharmaceutically acceptable salt thereof for a patient daily for 14 consecutive days.

In some embodiments, the therapeutic combination of an anti-PD-L1 antibody, and anlotinib or a pharmaceutically acceptable salt thereof described herein comprises: a pharmaceutical composition of the anti-PD-L1 antibody in which the anti-PD-L1 antibody is at a concentration of 10-60 mg/mL, and a pharmaceutical composition comprising 6 mg, 8 mg, 10 mg and/or 12 mg of anlotinib or the pharmaceutically acceptable salt thereof in a unit dose.

In some embodiments, the therapeutic combination or pharmaceutical composition comprising the anti-PD-L1 antibody and anlotinib or the pharmaceutically acceptable salt thereof disclosed herein comprises: a pharmaceutical composition comprising 1200 mg of the anti-PD-L1 antibody provided in multiple-dose form, and a pharmaceutical composition comprising 8 mg, 10 mg and/or 12 mg of anlotinib or the pharmaceutically acceptable salt thereof in a unit dose.

In some embodiments, the kit is suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib or the pharmaceutically acceptable salt thereof.

In some embodiments, provided is a method for treating non-MSI-H and/or non-dMMR endometrial cancer, which comprises: administering to a patient in need thereof a therapeutically effective amount of an anti-PD-L1 antibody, and anlotinib or a pharmaceutically acceptable salt thereof. In some embodiments, the anti-PD-L1 antibody and anlotinib or a pharmaceutically acceptable salt thereof are administered simultaneously, nonsimultaneously, or sequentially. In some embodiments, the anti-PD-L1 antibody is administered once every 1 week, every 2 weeks, every 3 weeks, or every 4 weeks. In some embodiments, the anti-PD-L1 antibody is administered at a dose of 600-2400 mg each time. In some embodiments, the anti-PD-L1 antibody is administered at a single dose of 600 mg, 800 mg, 1000 mg, 1200 mg, 1400 mg, 1600 mg, 1800 mg, 2000 mg, 2200 mg, or 2400 mg. In some embodiments, anlotinib or the pharmaceutically acceptable salt thereof is administered at a daily dose of 6 mg to 12 mg. In some embodiments, anlotinib or the pharmaceutically acceptable salt thereof is administered at a daily dose of 8 mg to 12 mg. In some embodiments, anlotinib or the pharmaceutically acceptable salt thereof is administered at a daily dose of 6 mg, 8 mg, 10 mg, or 12 mg. In some embodiments, the anlotinib or the pharmaceutically acceptable salt thereof is administered at a dose of 6 mg, 8 mg, 10 mg, or 12 mg once daily. In some embodiments, anlotinib or the pharmaceutically acceptable salt thereof is administered on a regimen of consecutively 2-week treatment and then 1-week interruption. In some embodiments, every 3 weeks are counted as one treatment cycle. In some embodiments, 21 days are counted as one treatment cycle, and the anti-PD-L1 antibody is administered to the patient on the first day of each treatment cycle. In some embodiments, every 3 weeks are counted as one treatment cycle, the anti-PD-L1 antibody is administered on the first day of each cycle, and anlotinib or the pharmaceutically acceptable salt thereof is administered on days 1-14 of each cycle. In some embodiments, the anti-PD-L1 antibody is administered parenterally. In some embodiments, the pharmaceutical composition of the anti-PD-L1 antibody has a concentration of 10-60 mg/mL; in some embodiments, the pharmaceutical composition of the anti-PD-L1 antibody has a concentration of 10 mg/mL, 20 mg/mL, 30 mg/mL, 40 mg/mL, 50 mg/mL, or 60 mg/mL. In some embodiments, anlotinib or the pharmaceutically acceptable salt thereof is administered orally.

In yet another aspect, the present application provides a method for treating endometrial cancer, which comprises: (1) obtaining or having obtained a biological sample of a patient; (2) testing or having tested the biological sample to determine whether the sample is MSI-H and/or dMMR; (3) administering to the patient a therapeutically effective amount of an anti-PD-L1 antibody or a pharmaceutical composition comprising the anti-PD-L1 antibody if the test result is MSI-H and/or dMMR; and (4) administering to the patient a therapeutically effective amount of an anti-PD-L1 antibody and anlotinib or a pharmaceutically acceptable salt thereof if the test result is non-MSI-H and/or non-dMMR.

In still another aspect, the present application provides a method for treating MSI-H and/or dMMR tumors, which comprises: (1) obtaining or having obtained a biological sample of a patient; (2) testing or having tested the biological sample to determine whether the sample is MSI-H or dMMR; and (3) administering to the patient a therapeutically effective amount of an anti-PD-L1 antibody or a pharmaceutical composition comprising the anti-PD-L1 antibody if the test result is MSI-H or dMMR.

Anlotinib or pharmaceutically acceptable salt thereof

The chemical name of anlotinib is 1-[[[4-(4-fluoro-2-methyl-1*H*-indol-5-yl)oxy-6-methoxyquinolin-7-yl]oxy]methyl]cyclopropylamine, which has the following structural formula:

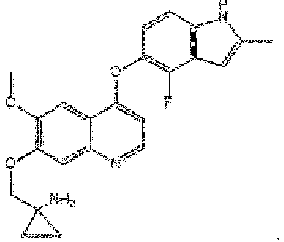

The pharmaceutically acceptable salts of anlotinib include, but are not limited to, salts formed from anlotinib and acids selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxynaphthoic acid, salicylic acid, and stearic acid. In some embodiments, the pharmaceutically acceptable salt is a hydrochloride or a maleate. In some embodiments, the pharmaceutically acceptable salt is a dihydrochloride.

Unless otherwise stated,the dose of anlotinib or the pharmaceutically acceptable salt thereof referred to in the present application is based on the molecular weight of the free base of anlotinib.

Anlotinib or the pharmaceutically acceptable salt thereof may be administered through various routes including gastrointestinal and parenteral including, but not limited to, oral, intravenous, intra-arterial, transdermal, sublingual, intramuscular, rectal, transbuccal, intranasal, inhalational, vaginal, intraocular, subcutaneous, intra-adipose, intra-articular, intraperitoneal, and intrathecal administrations. In some specific embodiments, the drug is administered orally. The amount of anlotinib or the pharmaceutically acceptable salt thereof administered can be determined according to the severity of the disease, the response of the disease, any treatment-related toxicity and the age and health of a patient. For example, the daily dose for administering anlotinib or the pharmaceutically acceptable salt thereof may be from 2 mg to 20 mg. In some embodiments, the daily dose for administering anlotinib or the pharmaceutically acceptable salt thereof may be 2 mg, 3 mg, 4 mg, 5 mg, 6 mg, 7 mg, 8 mg, 9 mg, 10 mg, 11 mg, 12 mg, 13 mg, 14 mg, 15 mg, and 16 mg. Anlotinib or the pharmaceutically acceptable salt thereof can be administered once or multiple times daily. In some embodiments, anlotinib or the pharmaceutically acceptable salt thereof is administered once daily in the form of a solid oral formulation.

The administration regimen of anlotinib or the pharmaceutically acceptable salt thereof can be determined depending on the combination of the activity and toxicity of the drug, and the tolerance of a patient, etc. Preferably, anlotinib or the pharmaceutically acceptable salt thereof is administered at intervals. The interval administration includes a treatment period and an interruption period. Anlotinib or the pharmaceutically acceptable salt thereof can be administered once or multiple times daily in the treatment period. For example, the ratio of the treatment period to the interruption period in days is 2:(0.5-5), 2:(0.5-3), 2:(0.5-2), or 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be orally administered at a dose of 6 mg, 8 mg, 10 mg or 12 mg once daily on a regimen of consecutively 2-week treatment and then 1-week interruption.

Pharmaceutical composition of anlotinib or pharmaceutically acceptable salt thereof

In some embodiments of the present application, a unit dose of the pharmaceutical composition of anlotinib or the pharmaceutically acceptable salt thereof comprises 6 mg, 8 mg, 10 mg or 12 mg of anlotinib.

In some embodiments of the present application, according to a treatment cycle of 2-week treatment and then 1-week interruption, a total dose of the pharmaceutical composition of anlotinib or the pharmaceutically acceptable salt thereof administered per cycle includes 84-168 mg. In some embodiments, a total dose of the pharmaceutical composition of anlotinib or the pharmaceutically acceptable salt thereof includes an amount selected from the group consisting of 84 mg, 112 mg, 140 mg and 168 mg or from a range formed of any of the aforementioned values. In some embodiments, a total dose of the pharmaceutical composition of anlotinib or the pharmaceutically acceptable salt thereof includes 112 mg to 168 mg.

In some embodiments, the pharmaceutical composition includes, but is not limited to, a formulation suitable for oral, and parenteral administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, tablets and capsules.

### Anti-PD-L1 antibody

In some embodiments, the anti-PD-L1 antibody is an anti-PD-L1 humanized monoclonal antibody.

In some embodiments of the present application, the anti-PD-L1 antibody is the antibody disclosed in WO2016022630 or CN107001463A.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, an isolated anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99% or 100%) homology to an amino acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the anti-PD-L1 humanized mAb disclosed herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, and SEQ ID NO: 21. The humanized anti-PD-L1 mAb comprising the conservatively substituted variants retains the ability to specifically recognize and bind to PD-L1.

In some embodiments of the present application, the anti-PD-L1 antibody may be an IgG1 or IgG4 antibody.

In some embodiments of the present application, the anti-PD-L1 antibody is an IgG1 antibody. In some embodiments, the anti-PD-L1 antibody is a glycosylated IgG1 antibody.

In some embodiments of the present application, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11. In one embodiment, the anti-PD-L1 antibody disclosed herein comprises: a heavy chain variable region selected from the group consisting of heavy chain variable regions of chimeric antibodies ch5G1-hIgG1, ch5G11-hIgG4, ch13C5-hIgG1, and ch13C5-hIgG4; and a light chain variable region selected from the group consisting of light chain variable regions of chimeric antibodies ch5G11-hIgG1, ch5G11-hIgG4, ch13C5-hIgG1, and ch13C5-hIgG4. In one embodiment, the anti-PD-L1 antibody disclosed herein comprises: a heavy chain variable region selected from the group consisting of heavy chain variable regions of humanized antibodies hu13C5-hIgG1, hu13C5-hIgG4, hu5G11-hIgG1, and hu5G11-hIgG4; and a light chain variable region selected from the group consisting of light chain variable regions of humanized antibodies hu13C5-hIgG1, hu13C5-hIgG4, hu5G11-hIgG1, and hu5G11-hIgG4. Reference can be made to the description of the patents WO2016022630 or CN107001463A: 13C5, ch13C5-hIgG1, ch13C5-hIgG4, hu13C5-hIgG1 or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ ID NO: 12); 5G11, ch5G11-hIgG1, ch5G11-hIgG4, hu5G11-hIgG1 or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9).

In some embodiments of the present application, the anti-PD-L1 antibody in the pharmaceutical combination may be selected from one or more. As used herein, the term "more" refers to more than one, for example, two, three, four, five or more. For example, in some embodiments of the present application, the anti-PD-L1 antibody is selected from the group consisting of an antibody comprising a heavy chain variable region set forth in SEQ ID NO: 13 and a light chain variable region set forth in SEQ ID NO: 15, or selected from the group consisting of an antibody comprising a heavy chain variable region set forth in SEQ ID NO: 14 and a light chain variable region set forth in SEQ ID NO: 16, or selected from the group consisting of a combination thereof. As another example, the anti-PD-L1 antibody is selected from the group consisting of an antibody comprising a heavy chain amino acid sequence set forth in SEQ ID NO: 17 and a light chain amino acid sequence set forth in SEQ ID NO: 18, or selected from the group consisting of an antibody comprising a heavy chain amino acid sequence set forth in SEQ ID NO: 19 and a light chain amino acid sequence set forth in SEQ ID NO: 20, or selected from the group consisting of an antibody comprising a heavy chain amino acid sequence set forth in SEQ ID NO: 21 and a light chain amino acid sequence set forth in SEQ ID NO: 18, or selected from the group consisting of combinations of any of the foregoing.

In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

### Pharmaceutical composition of anti-PD-L1 antibody

In some embodiments of the present application, the pharmaceutical composition of the anti-PD-L1 antibody comprises 600-2400 mg of the anti-PD-L1 antibody. In some embodiments, the pharmaceutical composition of the anti-PD-L1 antibody comprises the anti-PD-L1 antibody includes an amount selected from the group consisting of 600 mg, 900 mg, 1200 mg, 1500 mg, 1800 mg, 2100 mg and 2400 mg or from a range formed of any of the aforementioned values. In some embodiments, the pharmaceutical composition of the anti-PD-L1 antibody comprises 600-2100 mg or 900-1500 mg of the anti-PD-L1 antibody; wherein the pharmaceutical composition of the anti-PD-L1 antibody may be present in multiple-dose or in unit-dose form.

In some embodiments of the present application, the pharmaceutical composition of the anti-PD-L1 antibody comprises 300 mg, 600 mg, or 1200 mg of the anti-PD-L1 antibody. In some embodiments of the present application, provided is a pharmaceutical composition formulated as a unit dose of an anti-PD-L1 antibody, which comprises 300 mg, 600 mg, or 1200 mg of the anti-PD-L1 antibody.

In some embodiments of the present application, the pharmaceutical composition of the anti-PD-L1 antibody is a solution for injection. In some embodiments of the present application, the pharmaceutical composition of the anti-PD-L1 antibody is aqueous solution for injection. In some embodiments of the present application, the pharmaceutical composition of the anti-PD-L1 antibody comprises one or more of a buffer, an isotonicity modifier, a stabilizer and/or a surfactant. In particular, the pharmaceutical composition of the anti-PD-L1 antibody comprises 1-150 mg/mL anti-PD-L1 antibody (e.g., mAb), 3-50 mM buffer, 2-150 mg/mL isotonicity adjusting agent/stabilizer, and 0.01-0.8 mg/mL surfactant, with a pH of 4.5-6.8.

In some embodiments of the present application, in the pharmaceutical composition of the anti-PD-L1 antibody, the anti-PD-L1 mAb is at a concentration of 5-150 mg/mL (w/v); in some embodiments, the anti-PD-L1 mAb is at a concentration of 10-60 mg/mL (w/v); in some embodiments, the anti-PD-L1 mAb is at a concentration of 10-30 mg/mL (w/v). In some embodiments, the anti-PD-L1 mAb is at a concentration of 10 mg/mL (w/v), 20 mg/mL (w/v), 30 mg/mL (w/v), 40 mg/mL (w/v), 50 mg/mL (w/v), 60 mg/mL (w/v), 70 mg/mL (w/v), 80 mg/mL (w/v), 90 mg/mL (w/v), 100 mg/mL (w/v), 110 mg/mL (w/v), or 120 mg/mL (w/v); in some embodiments, the anti-PD-L1 mAb is at a concentration of 10 mg/mL (w/v), 20 mg/mL (w/v), 30 mg/mL (w/v), 40 mg/mL (w/v), 50 mg/mL (w/v), or 60 mg/mL (w/v); in some embodiments, the anti-PD-L1 mAb is at a concentration of 10 mg/mL (w/v), 20 mg/mL (w/v), or 30 mg/mL (w/v). In some embodiments, the anti-PD-L1 mAb is at a concentration of 10 mg/mL (w/v). In other embodiments, the anti-PD-L1 mAb is at a concentration of 30 mg/mL (w/v). In other embodiments, the anti-PD-L1 mAb is at a concentration of 60 mg/mL (w/v).

In some embodiments of the present application, the buffer is a histidine salt buffer. The histidine salt buffer is at a concentration of 5-30 mM; in some embodiments, the histidine salt buffer is at a concentration of 10-25 mM; in some embodiments, the histidine salt buffer is at a concentration of 10-20 mM; in some embodiments, the histidine salt buffer is at a concentration of 10-15 mM. In some embodiments, the histidine salt buffer is at a concentration of 5 mM, 10 mM, 15 mM, 20 mM, 25 mM, or 30 mM. In some embodiments, the histidine salt buffer is at a concentration of 10 mM. In other embodiments, the histidine salt buffer is at a concentration of 15 mM. In other embodiments, the histidine salt buffer is at a concentration of 20 mM. The histidine salt buffer comprises histidine and hydrochloric acid.

In some embodiments of the present application, the isotonicity modifier/stabilizer is sucrose at 20-150 mg/mL (w/v); in some embodiment, the isotonicity modifier/stabilizer is sucrose at 40-100 mg/mL (w/v); in some embodiment, the isotonicity modifier/stabilizer is sucrose at 60-80 mg/mL (w/v). In some specific embodiments, the sucrose is at a concentration of 40 mg/mL, 50 mg/mL, 60 mg/mL, 70 mg/mL, 80 mg/mL, 90 mg/mL or 100 mg/mL. In some specific embodiments, the sucrose is at a concentration of 60 mg/mL. In some specific embodiments, the sucrose is at a concentration of 70 mg/mL. In some specific embodiments, the sucrose is at a concentration of 80 mg/mL. In some specific embodiments, the sucrose is at a concentration of 90 mg/mL.

In some embodiments of the present application, the surfactant is selected from the group consisting of polysorbate 80, polysorbate 20 and poloxamer 188; in some embodiments, the surfactant is selected from the group consisting of polysorbate 80 and polysorbate 20; in some embodiments, the surfactant is selected from the group consisting of polysorbate 80. In some embodiments, the surfactant is at a concentration of 0.05-0.6 mg/mL (w/v); in some embodiments, the surfactant is at a concentration of 0.1-0.4 mg/mL (w/v); in some embodiments, the surfactant is at a concentration of 0.2-0.3 mg/mL (w/v).

In some embodiments of the present application, the surfactant is polysorbate 80 or polysorbate 20 at 0.01-0.8 mg/mL (w/v). In some specific embodiments, the surfactant is polysorbate 80 at 0.05-0.6 mg/mL; in some embodiments, the surfactant is polysorbate 80 at 0.1-0.4 mg/mL; in some embodiments, the surfactant is polysorbate 80 at 0.2-0.3 mg/mL; in some embodiments, the surfactant is polysorbate 80 at 0.2 mg/mL. In some embodiments, the pharmaceutical composition has a polysorbate 80 content of 0.1 mg/mL, 0.2 mg/mL, 0.3 mg/mL, 0.4 mg/mL, 0.5 mg/mL, or 0.6 mg/mL. In some embodiments, the pharmaceutical composition has a polysorbate 80 content of 0.2 mg/mL, 0.3 mg/mL, 0.4 mg/mL, or 0.5 mg/mL. In some embodiments, the pharmaceutical composition has a polysorbate 80 content of 0.2 mg/mL, 0.3 mg/mL, or 0.4 mg/mL. In some embodiments, the pharmaceutical composition has a polysorbate 80 content of 0.2 mg/mL. In some embodiments, the pharmaceutical composition has a polysorbate 80 content of 0.1 mg/mL. In other embodiments, the pharmaceutical composition has a polysorbate 80 content of 0.2 mg/mL. In some embodiments, the pharmaceutical composition has a polysorbate 80 content of 0.3 mg/mL. In other embodiments, the pharmaceutical composition has a polysorbate 80 content of 0.4 mg/mL. In some embodiments, the pharmaceutical composition has a polysorbate 80 content of 0.5 mg/mL.

In some embodiments of the present application, an aqueous solution of the pharmaceutical composition has a pH selected from 4.0-6.8; in some embodiments, an aqueous solution of the pharmaceutical composition has a pH of 4.5-6.5; in some embodiments, an aqueous solution of the pharmaceutical composition has a pH of 5.5-6.0; in some embodiments, an aqueous solution of the pharmaceutical composition has a pH of 5.5. In some embodiments, an aqueous solution of the pharmaceutical composition has a pH of 4.5, 4.8, 5.0, 5.2, 5.4, 5.5, 5.6, 5.8, or 6.0; in some embodiments, an aqueous solution of the pharmaceutical composition has a pH of 5.0, 5.2, 5.4, 5.5, or 5.6; in some embodiments, an aqueous solution of the pharmaceutical composition has a pH of 5.5. In some embodiments, an aqueous solution of the pharmaceutical composition has a pH of 5.0. In some embodiments, an aqueous solution of the pharmaceutical composition has a pH of 5.2. In some embodiments, an aqueous solution of the pharmaceutical composition has a pH of 5.4. In some embodiments, an aqueous solution of the pharmaceutical composition has a pH of 5.5. In some embodiments, an aqueous solution of the pharmaceutical composition has a pH of 5.6. In some embodiments, an aqueous solution of the pharmaceutical composition has a pH of 5.8. In some embodiments, an aqueous solution of the pharmaceutical composition has a pH of 6.0.

In some specific embodiments of the present application, the pharmaceutical composition comprises: (a) an anti-PD-L1 antibody at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0. In one specific embodiment of the present application, the pharmaceutical composition comprises: (a) an anti-PD-L1 mAb at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0.

In another specific embodiment of the present application, the pharmaceutical composition comprises: (a) an anti-PD-L1 antibody at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 50 mg/mL (w/v), (b) sucrose at a concentration of 80 mg/mL (w/v), (c) polysorbate 80 at a concentration of 0.3 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 100 mg/mL (w/v), (b) sucrose at a concentration of 80 mg/mL (w/v), (c) polysorbate 80 at a concentration of 0.5 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 30 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 60 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 10 mg/mL (w/v), (b) sucrose at a concentration of 70 mg/mL (w/v), (c) polysorbate 80 at a concentration of 0.4 mg/mL (w/v), (d) histidine at a concentration of 20 mM, and (e) optionally a suitable amount of acetic acid for adjusting the pH of the composition to 6.5.

In another specific embodiment of the present application, the pharmaceutical composition comprises: (a) an anti-PD-L1 mAb at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 20 mM, and (e) optionally a suitable amount of hydrochloric acid for adjusting the pH of the composition to 5.5.

In another specific embodiment of the present application, the pharmaceutical composition is a water-soluble injection. In some embodiments, the water-soluble injection includes, but is not limited to, a water-soluble formulation without lyophilization or a water-soluble formulation reconstituted from a lyophilized powder. In other embodiments, the pharmaceutical composition is a lyophilized formulation. The lyophilized formulation refers to a formulation prepared by subjecting an aqueous solution to a lyophilization process in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulations of the present application can also be dried by other methods known in the art, such as spray drying and bubble drying.

### Endometrial cancer

In the present application, the endometrial cancer includes, but is not limited to, type I (estrogen-dependent) endometrial cancer or type II (estrogen-independent) endometrial cancer.

In some embodiments of the present application, the endometrial cancer is advanced endometrial cancer. In some embodiments of the present application, the endometrial cancer is refractory endometrial cancer.

In some embodiments of the present application, the endometrial cancer is recurrent and/or metastatic endometrial cancer.

In some embodiments of the present application, the endometrial cancer is recurrent and/or metastatic advanced endometrial cancer.

In some embodiments of the present application, the non-microsatellite instability-High mutation (non-MSI-H) endometrial cancer includes microsatellite instability-low (MSI-L) endometrial cancer and microsatellite stability (MSS) endometrial cancer.

In some embodiments of the present application, the non-deficient mismatch repair (non-dMMR) endometrial cancer comprises proficient mismatch repair (pMMR) endometrial cancer.

In some embodiments of the present application, the patient with endometrial cancer has received one or more drug therapies. In some embodiments of the present application, the endometrial cancer is endometrial cancer that has failed at least one drug therapy. In some embodiments, the treatment failure comprises disease progression during treatment, and disease progression and/or recurrence after treatment.

In some embodiments of the present application, the patient with endometrial cancer has received one or more chemotherapies. In some embodiments of the present application, the endometrial cancer is endometrial cancer that has failed at least one chemotherapy treatment. In some embodiments, the treatment failure comprises disease progression or lack of objective response during treatment, and disease progression and/or recurrence after treatment. In some embodiments of the present application, the patient with endometrial cancer has previously received 1-2 lines of systemic standard chemotherapy regimen, and the treatment fails or is intolerant.

In the present application, the drug therapy includes, but is not limited to, chemotherapy, targeted drug therapy, and hormone therapy.

In some embodiments of the present application, the chemotherapy comprises, but is not limited to, one or more of taxanes, vinca alkaloid antineoplastic agents, platinum complexes, fluorouracil and derivatives thereof, camptothecin and derivatives thereof, anthracycline compounds, and podophyllum compounds. The taxanes include, but are not limited to, one or more of paclitaxel, albumin-bound paclitaxel, and docetaxel; the vinca alkaloid antineoplastic agents include, but are not limited to, one or more of vinblastine, vincristine, vindesine, vinorelbine, vinflunine and navelbine; the platinum complexs include, but are not limited to, one or more of miriplatin, cisplatin, carboplatin, dicycloplatin, nedaplatin, oxaliplatin, lobaplatin, triplatin tetranitrate, phenanthriplatin, picoplatin, and satraplatin; the luorouracil and derivatives thereof include, but are not limited to, one or more of cytarabine, azacitidine, ancitabine, capecitabine, gemcitabine, fluorouracil, difuradin, doxifluridine, trifluridine, tegafur, carmofur and tegafur; the camptothecin and derivatives thereof include, but are not limited to, one or more of camptothecin, hydroxycamptothecin, irinotecan, and topotecan; the anthracycline compounds include, but are not limited to, one or more of epirubicin, doxorubicin, daunorubicin, pirarubicin, amrubicin, idarubicin, mitoxantrone, aclarubicin, valrubicin, zorubicin, pixantrone, Pirarubicin, and liposomal doxorubicin; the podophyllum compounds include, but are not limited to, one or more of etoposide, teniposide, and etoposide.

In some embodiments of the present application, the targeted drug includes, but is not limited to, one or more of an EGFR antagonist, a VEGF inhibitor, an HER2 inhibitor, a PARP inhibitor, a PI3K/Akt/mTOR pathway inhibitor, a PD-1/PD-L1 inhibitor, or an FGFR inhibitor.

In some embodiments of the present application, the EGFR antagonist includes, but is not limited to, one or more of trastuzumab, cetuximab, icotinib, gefitinib, erlotinib, and lapatinib. In some embodiments of the present application, the VEGF inhibitor includes, but is not limited to, bevacizumab, ranibizumab, axitinib, motesanib, aflibercept, cediranib, nintedanib, sorafenib, or sunitinib. In some embodiments of the present application, the PARP inhibitor includes, but is not limited to, one or more of olaparib, niraparib or rucaparib. In some embodiments of the present application, the PI3K/Akt/mTOR pathway inhibitor includes, but is not limited to, one or more of NVP-BKM120, XL147, perifosine, rapamycin, temsirolimus, everolimus, sirolimus, or ridaforolimus. In some embodiments of the present application, the PD-1/PD-L1 inhibitor includes, but is not limited to, one or more of atezofizumab, nivolumab, or pembrolizumab. In some embodiments of the present application, the FGFR inhibitor includes, but is not limited to, dovitinib or NVP-BGJ398.

In some embodiments of the present application, the hormone-therapeutic drug includes, but is not limited to, one or more of a progestogen, an anti-estrogen, and an aromatase inhibitor. In some embodiments of the present application, the progestin drug includes, but is not limited to, one or more of progesterone, megestrol acetate, chlormadinone, medroxyprogesterone acetate, hydroxyprogesterone acetate, hydroxyprogesterone caproate, cyproterone acetate, dydrogesterone, demegestone, promegestone, lynestrenol, norethindrone, levogestrel, desogestrel, dienogest, or drospirenone. In some embodiments of the present application, the anti-estrogen includes, but is not limited to, one or more of clomiphene, tamoxifen, toremifene, raloxifene, triptorelin, leuprorelin, or goserelin. In some embodiments of the present application, the aromatase inhibitor includes, but is not limited to, one or more of aminoglutethimide, formestane, exemestane, letrozole, anastrozole, rogletimide or fadrozole.

### MSI-H and/or dMMR tumor

In some embodiments, the tumor is a solid tumor; in some embodiments, the tumor is a malignant tumor; in some embodiments, the tumor is a malignant solid tumor.

The MSI-H and/or dMMR tumor described herein is based on biomarker detection, e.g., as shown in MSI-H and/or dMMR, without limitation to the type of tumor.

In the present application, the solid tumor does not include a hematological/blood tumor.

In some embodiments, the tumor includes, but is not limited to, one or more of breast cancer (e.g., hormone receptor positive breast cancer, triple-negative breast cancer, HER2/neu positive or negative breast cancer), digestive system /gastrointestinal tumors (e.g., anal cancer, appendiceal cancer, biliary cancer, gastrointestinal stromal tumor, gastrointestinal carcinoid, colorectal cancer, gastric cancer, esophageal cancer, gallbladder cancer, bile duct cancer, liver cancer, pancreatic cancer, tumors of pancreatic islet cell tumor, pancreatic neuroendocrine tumor), endocrine system tumor and neuroendocrine cancer (e.g. adrenocortical carcinoma, thyroid cancer, cutaneous neuroendocrine tumor, parathyroid carcinoma), eye cancer, genitourinary system cancer (e.g., bladder cancer, kidney cancer, prostate cancer, testicular cancer, urethral cancer, urothelial cancer), gynecological cancer (e.g., endometrial cancer, ovarian cancer, cervical cancer, fallopian tube cancer, primary peritoneal cancer, vaginal cancer, vulvar cancer), head and neck cancer (e.g., hypopharyngeal cancer, laryngeal cancer, lip and oral cancer, nasopharyngeal cancer, oropharyngeal cancer, paranasal sinus and nasal cavity cancer, pharyngeal cancer, salivary gland cancer, pharyngeal cancer), respiratory system cancer (e.g., lung cancer (including but not limited to non-small cell lung cancer, small cell lung cancer), malignant mesothelioma, thymus cancer), skin cancer (e.g., Merkel cell carcinoma, squamous cell skin carcinoma, melanoma), nervous system tumor (e.g., brain cancer, glioblastoma, neuroblastoma, pituitary tumor, primary central nervous system lymphoma), bone cancer, soft tissue sarcoma, and hematologic/blood tumors (e.g., leukemia, lymphoma).

In some embodiments, the malignant tumor includes, but is not limited to, one or more of breast cancer (e.g., hormone receptor positive breast cancer, triple-negative breast cancer, HER2/neu positive or HER2/neu negative breast cancer), digestive system cancer/gastrointestinal cancer (e.g., anal cancer, appendiceal cancer, biliary cancer, gastrointestinal stromal tumor, gastrointestinal carcinoid, colorectal cancer, gastric cancer, esophageal cancer, gallbladder cancer, bile duct cancer, liver cancer, pancreatic cancer), endocrine system cancer and neuroendocrine cancer (e.g. adrenocortical carcinoma, thyroid cancer, cutaneous neuroendocrine tumor, parathyroid carcinoma), eye cancer, genitourinary system cancer (e.g., bladder cancer, kidney cancer, prostate cancer, testicular cancer, urethral cancer, urothelial cancer), gynecological cancer (e.g., endometrial cancer, ovarian cancer, cervical cancer, fallopian tube cancer, primary peritoneal cancer, vaginal cancer, vulvar cancer), head and neck cancer (e.g., hypopharyngeal cancer, laryngeal cancer, lip and oral cancer, nasopharyngeal cancer, oropharyngeal cancer, paranasal sinus cancer and nasal cavity cancer, pharyngeal cancer, salivary gland cancer, pharyngeal cancer), respiratory system cancer (e.g., lung cancer (including but not limited to non-small cell lung cancer, small cell lung cancer), malignant mesothelioma, thymus cancer), skin cancer (e.g., Merkel cell carcinoma, squamous cell skin carcinoma, melanoma), nervous system malignant tumor (e.g., brain cancer, glioblastoma, neuroblastoma, primary central nervous system lymphoma), bone cancer, soft tissue sarcoma, and hematologic/blood malignant tumor (hematologic/blood cancer) (e.g., leukemia, lymphoma).

In some embodiments of the present application, the MSI-H and/or dMMR tumor is cervical cancer (including but not limited to cervical squamous carcinoma, cervical adenocarcinoma). In some embodiments of the present application, the MSI-H and/or dMMR tumor is hepatobiliary system cancer (including but not limited to liver cancer, gallbladder cancer, bile duct cancer). In some embodiments of the present application, the MSI-H and/or dMMR tumor is liver cancer (including but not limited to hepatocellular carcinoma, intrahepatic cholangiocarcinoma, extrahepatic cholangiocarcinoma). In some embodiments of the present application, the MSI-H and/or dMMR tumor is urothelial cancer (including but not limited to bladder cancer, urethral cancer). In some embodiments of the present application, the MSI-H and/or dMMR tumor is lung cancer (e.g., small cell lung cancer, non-small cell lung cancer (including but not limited to lung squamous cell carcinoma, lung adenocarcinoma)). In some embodiments of the present application, the MSI-H and/or dMMR tumor is leukemia (including but not limited to acute myeloid leukemia, chronic myeloid leukemia, chronic lymphocytic leukemia). In some embodiments of the present application, the MSI-H and/or dMMR tumor is lymphoma (including but not limited to Hodgkin's lymphoma, primary mediastinal large B-cell lymphoma). In some embodiments of the present application, the MSI-H and/or dMMR tumor is colorectal cancer.

In some embodiments of the present application, the tumor is an advanced and/or metastatic tumor. In some embodiments of the present application, the tumor is a refractory and/or recurrent tumor.

In some embodiments of the present application, the MSI-H and/or dMMR malignant tumor is an advanced MSI-H and/or dMMR malignant tumor. In some embodiments of the present application, the MSI-H and/or dMMR malignant tumor is a refractory MSI-H and/or dMMR malignant tumor.

In some embodiments of the present application, the MSI-H and/or dMMR malignant tumor is a recurrent and/or metastatic MSI-H and/or dMMR malignant tumor.

In some embodiments of the present application, the MSI-H and/or dMMR malignant tumor is a recurrent and/or metastatic advanced MSI-H and/or dMMR malignant tumor.

In some embodiments of the present application, the MSI-H and/or dMMR solid tumor includes, but is not limited to, one or more of breast cancer (e.g., hormone receptor positive breast cancer, triple-negative breast cancer, HER2/neu positive or negative breast cancer), digestive system /gastrointestinal tumors (e.g., anal cancer, appendiceal cancer, biliary cancer, gastrointestinal stromal tumor, gastrointestinal carcinoid, colorectal cancer, gastric cancer, esophageal cancer, gallbladder cancer, bile duct cancer, liver cancer, pancreatic cancer, tumors of pancreatic islet cell tumor, pancreatic neuroendocrine tumor), endocrine system tumor and neuroendocrine cancer (e.g., adrenocortical carcinoma, thyroid cancer, cutaneous neuroendocrine tumor, parathyroid carcinoma), eye cancer, genitourinary system cancer (e.g., bladder cancer, kidney cancer, prostate cancer, testicular cancer, urethral cancer, urothelial cancer), gynecological cancer (e.g., endometrial cancer, ovarian cancer, cervical cancer, fallopian tube cancer, primary peritoneal cancer, vaginal cancer, vulvar cancer), head and neck cancer (e.g., hypopharyngeal cancer, laryngeal cancer, lip and oral cancer, nasopharyngeal cancer, oropharyngeal cancer, paranasal sinus and nasal cavity cancer, pharyngeal cancer, salivary gland cancer, pharyngeal cancer), respiratory system cancer (e.g., lung cancer (including but not limited to non-small cell lung cancer, small cell lung cancer), malignant mesothelioma, thymus cancer), skin cancer (e.g., Merkel cell carcinoma, squamous cell skin carcinoma, melanoma), nervous system tumor (e.g., brain cancer, glioblastoma, neuroblastoma, pituitary tumor, primary central nervous system lymphoma), bone cancer, and soft tissue sarcoma.

In some embodiments of the present application, the MSI-H and/or dMMR solid tumor is a malignant solid tumor. In some embodiments of the present application, the MSI-H and/or dMMR malignant solid tumor is an advanced malignant solid tumor. In some embodiments of the present application, the MSI-H and/or dMMR malignant solid tumor is a refractory malignant solid tumor.

In some embodiments of the present application, the MSI-H and/or dMMR malignant solid tumor is a recurrent and/or metastatic malignant solid tumor.

In some embodiments of the present application, the MSI-H and/or dMMR malignant solid tumor is a recurrent and/or metastatic advanced malignant solid tumor.

In some embodiments of the present application, the patient with the MSI-H and/or dMMR tumor has received one or more prior therapies. In some embodiments of the present application, the MSI-H and/or dMMR tumor is an MSI-H and/or dMMR tumor that has undergone disease progression after prior therapy and/or has not had satisfactory alternative therapy.

In some embodiments of the present application, the patient with the MSI-H and/or dMMR tumor has received one or more drug therapies. In some embodiments of the present application, the MSI-H and/or dMMR tumor is an MSI-H and/or dMMR tumor that has failed at least one drug therapy. In some embodiments, the treatment failure comprises disease progression during treatment, and disease progression and/or recurrence after treatment. In some embodiments of the present application, the patient with the MSI-H and/or dMMR tumor has received one or more chemotherapies. In some embodiments of the present application, the MSI-H and/or dMMR tumor is an MSI-H and/or dMMR tumor that has failed at least one chemotherapeutic therapy. In some embodiments, the treatment failure comprises disease progression during treatment, and disease progression and/or recurrence after treatment. In some embodiments of the present application, the patient with the MSI-H and/or dMMR tumor has previously received a 1-2 line systemic standard chemotherapy regimen, and the treatment fails or is intolerant.

Regimen for anti-PD-L1 antibody or pharmaceutical compositions thereof

In some embodiments of the present application, in the use or treatment method described above, the anti-PD-L1 antibody can be administered once every week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at a dose of 600-2400 mg each time.

In some embodiments of the present application, in the use or treatment method, 21 days are counted as one treatment cycle, and the anti-PD-L1 antibody is administered on the first day of each cycle.

In some embodiments of the present application, in the use or treatment method, the anti-PD-L1 antibody can be administered to a subject at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, or 15 to 20 mg/kg, or administered to a subject at a dose of 60 mg to 2400 mg, 90 mg to about 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments of the use or treatment method, 21 days are counted as one treatment cycle, and 1200 mg of the PD-L1 antibody is administered on the first day of each cycle.

### Administration mode of anti-PD-L1 antibody or pharmaceutical composition thereof

The content below is not intended to limit the administration mode of the antibody or the pharmaceutical composition thereof disclosed herein.

The anti-PD-L1 antibody and the pharmaceutical composition thereof disclosed herein may be administered by a suitable route, including but not limited to, oral or parenteral route (e.g., by intravenous, intramuscular, local, or subcutaneous routes). In some embodiments, the antibody and the pharmaceutical composition thereof disclosed herein is administered by injection, for example, intravenous injection or intraperitoneal injection.

The pharmaceutical composition disclosed herein may be in dosage form, including but not limited to, tablet, lozenge, pill, capsule (e.g., hard capsule, soft capsule, enteric capsule and microcapsule), elixir, granule, syrup, injection (intramuscular, intravenous and intraperitoneal), granule, emulsion, suspension, solution, dispersant and dosage forms of sustained release formulations for oral or non-oral administration.

The pharmaceutical composition disclosed herein may further comprise a pharmaceutically acceptable carrier and/or excipient.

### Regimen for therapeutic combination

In some embodiments of the present application, in the use or treatment method described above, the anti-PD-L1 antibody and anlotinib or the pharmaceutically acceptable salt thereof are each in the form of a pharmaceutical composition and can be administered simultaneously, nonsimultaneously, or sequentially.

In some embodiments of the present application, in the use or treatment method described above, the anti-PD-L1 antibody and anlotinib are separately administered at intervals. In some embodiments, the antibody and anlotinib are administered separately on the same regimen or different regimens. In some embodiments, the two are separately administered on different regimens.

In some embodiments of the present application, in the use or treatment method described above, the anti-PD-L1 antibody can be administered once every 1 week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at a dose of 600-2400 mg each time. Anlotinib or the pharmaceutically acceptable salt thereof can be administered at a dose of 6 mg, 8 mg, 10 mg or 12 mg once daily on a regimen of consecutively 2-week treatment and then 1-week interruption.

In some embodiments, the anti-PD-L1 antibody and anlotinib or the pharmaceutically acceptable salt thereof are used in identical or different treatment cycles. In some specific embodiments, the anti-PD-L1 antibody and anlotinib are used in identical treatment cycles, e.g., in 1-week, 2-week, 3-week or 4-week treatment cycles.

In some embodiments of the present application, in the use or treatment method, one treatment cycle comprises 21 days; the PD-L1 antibody is administered on the first day of each treatment cycle, and anlotinib or the pharmaceutically acceptable salt thereof is administered daily on days 1-14 of each cycle. In one specific embodiment, the PD-L1 antibody is administered once on the first day of each treatment cycle, and anlotinib or the pharmaceutically acceptable salt thereof is administered once daily on days 1-14 of each cycle.

In some embodiments of the present application, in the use or treatment method, the anti-PD-L1 antibody can be administered to a subject at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, or 15 to 20 mg/kg, or administered to a subject at a dose of 60 mg to 2400 mg, 90 mg to 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments for the use or treatment method, 21 days are counted as one treatment cycle, 1200 mg of the PD-L1 antibody is administered on the first day of each treatment cycle, and 6 mg, 8 mg, 10 mg and/or 12 mg of anlotinib or the pharmaceutically acceptable salt thereof is administered daily on days 1-14 of each cycle.

In some embodiments of the present application, every three weeks are counted as one treatment cycle, the anti-PD-L1 antibody and anlotinib or the pharmaceutically acceptable salt thereof are administered to a subject in a weight ratio of (0.35-29):1, (3.5-29):1, (3.5-14.5):1, or (7-14.5):1, wherein the anti-PD-L1 antibody and anlotinib or the pharmaceutically acceptable salt thereof are administered in a unit dose and multiple doses, respectively.

### Administration mode of therapeutic combination

The content below is not intended to limit the administration of the pharmaceutical combination disclosed herein. The components in the pharmaceutical combination disclosed herein can be administered independently, or some or all of the components are co-administered in various proper routes, including, but not limited to, oral administration or parenteral administration (by intravenous, intramuscular, local or subcutaneous routes). In some embodiments, the components in the pharmaceutical combination disclosed herein can be administered independently, or some or all of the components are co-administered by means of oral administration or injection, for example, intravenous injection or intraperitoneal injection.

The components of the pharmaceutical combination of the present application can be independent suitable dosage forms, or some or all of the components are co-formulated in a suitable dosage form including but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-release formulations for oral or non-oral administration.

The components in the pharmaceutical combination disclosed herein can be formulated independently, or some or all of the components are co-formulated with a pharmaceutically acceptable carrier and/or excipient.

The anti-PD-L1 antibody disclosed herein can safely and effectively treat endometrial cancer.

In some embodiments, the anti-PD-L1 antibody disclosed herein shows greater sensitivity to MSI-H or dMMR endometrial cancer, and the objective response rate (ORR) in patients with MSI-H or dMMR endometrial cancer is far greater than 15% after treatment with the anti-PD-L1 antibody; in a specific embodiment, the ORR is up to or greater than 30%. In some embodiments, the disease control rate (DCR) of the patient is up to or greater than 50% after receiving treatment with the anti-PD-L1 antibody. In a specific embodiment, the DCR is up to or greater than 80%.

In a specific embodiment, the anti-PD-L1 antibody provided herein treats MSI-H or dMMR endometrial cancer, which can significantly reduce the incidence and severity of treatment-related adverse events (TRAEs), wherein an incidence of treatment-related adverse events is less than 80%, and an incidence of treatment-related adverse events above grade 3 is less than 20%. In some embodiments, the anti-PD-L1 antibody provided herein treats MSI-H or dMMR endometrial cancer with an incidence of treatment-related adverse events of less than 70% and an incidence of treatment-related adverse events above grade 3 of no more than 16%.

The anti-PD-L1 antibody disclosed herein shows a higher sensitivity to MSI-H and/or dMMR tumors, e.g. MSI-H and/or dMMR malignant tumors, and can safely and effectively treat MSI-H and/or dMMR tumors with a patient objective response rate (ORR) of far greater than 15%. In some specific embodiments, the objective response rate (ORR) for MSI-H and/or dMMR tumors, e.g., MSI-H or dMMR endometrial cancer, in a patient after treatment with an anti-PD-L1 antibody is far in excess of 15%. In some specific embodiments, the ORR is up to or greater than 30%. In some embodiments, the disease control rate (DCR) of the patient is up to or greater than 50% after treatment with the anti-PD-L1 antibody. In some specific embodiments, the DCR is up to or greater than 80%.

In some embodiments, the anti-PD-L1 antibody provided herein treats MSI-H or dMMR tumor, which can significantly reduce the incidence and severity of treatment-related adverse events (TRAEs), wherein an incidence of treatment-related adverse events is less than 80%, and an incidence of treatment-related adverse events above grade 3 is less than 20%. In some embodiments, the anti-PD-L1 antibody provided herein treats MSI-H or dMMR malignant tumor with an incidence of treatment-related adverse events of less than 70% and an incidence of treatment-related adverse events above grade 3 of no more than 16%.

In some embodiments, the therapeutic combination comprising an anti-PD-L1 antibody and anlotinib or a pharmaceutically acceptable salt thereof disclosed herein can safely and effectively treat endometrial cancer.

In some embodiments, the therapeutic combination comprising an anti-PD-L1 antibody and anlotinib or a pharmaceutically acceptable salt thereof disclosed herein can safely and effectively treat non-MSI-H and/or non-dMMR endometrial cancer. In some embodiments, the therapeutic combination comprising an anti-PD-L1 antibody, and anlotinib or a pharmaceutically acceptable salt thereof disclosed herein has an ORR (objective response rate) of greater than 15% in patients with non-MSI-H and/or non-dMMR endometrial cancer. In a specific embodiment, the ORR is up to or greater than 25%. In a specific embodiment, the ORR is up to or greater than 30%. The disease control rate (DCR) is up to or greater than 80%. In some specific embodiments, the DCR is up to or greater than 90%.

In some embodiments, the anti-PD-L1 antibody and anlotinib or the pharmaceutically acceptable salt thereof disclosed herein have a synergistic effect in treating endometrial cancer. In some embodiments, the anti-PD-L1 antibody and anlotinib or the pharmaceutically acceptable salt thereof disclosed herein have a significant synergistic effect in treating non-MSI-H and/or non-dMMR endometrial cancer.

In some embodiments, the anti-PD-L1 antibody and anlotinib or the pharmaceutically acceptable salt thereof provided treat endometrial cancer, especially non-MSI-H and/or non-dMMR endometrial cancer, which can significantly reduce the incidence and severity of treatment-related adverse events (TRAEs), wherein an incidence of treatment-related adverse events is less than 80%, and an incidence of treatment-related adverse events above grade 3 is less than 10%. In some embodiments, the anti-PD-L1 antibody and anlotinib or the pharmaceutically acceptable salt thereof provided herein treat non-MSI-H and/or non-dMMR endometrial cancer with an incidence of treatment-related adverse events of no more than 75% and an incidence of treatment-related adverse events above grade 3 of no more than 7%.

In some embodiments, ORR is calculated according to the ratio of the number of objective response cases (PR + CR) evaluated by IRC to the total number of cases and 95% CI.

In some embodiments of the present application, the immunogenicity of a patient is studied; the immunogenicity study includes the study of the anti-drug antibody ADA and the neutralizing antibody Nab, and if the patient's anti-drug antibody (ADA) is detected to be positive, the neutralizing antibody (Nab) is added. In some embodiments, the time point for immunogenicity monitoring is based on the time of administration of the antibody injection; when the antibody administration is delayed, immunogenic blood sampling is delayed accordingly.

In the present application, MSI (microsatellite instability) is caused by loss of function of a DNA mismatch repair (MMR) protein. In the present application, MSI status may be detected according to methods well known in the art, and methods for detecting MSI status include, but are not limited to, immunohistochemical detection of MMR proteins, multiplex fluorescent polymerase chain reaction PCR detection of microsatellite loci, and MSI detection based on a next generation sequencing (NGS) platform. In a specific embodiment, MSI is detected using multiplex fluorescent polymerase chain reaction PCR. The status of MSI is detected, for example, using commercially available kits. In some embodiments, according to multiplex fluorescent polymerase chain reaction PCR detection of microsatellite loci, the interpretation standard changes with the number of kit loci, and the status of MSI, such as MSI-H (microsatellite instability-high), MSI-L (microsatellite instability-low), and MSS (microsatellite stability), is determined according to the criteria for determination of commercially available qualified kits.

In the present application, the status of MMR (e.g., dMMR) can be detected according to methods well known in the art, for example, the MMR proteins can be detected using immunohistochemistry (IHC), for example, the four MMR proteins (MLH1, MSH2, MSH6 and PMS2) in a tumor sample of a patient can be detected primarily, wherein a deletion of any MMR protein is dMMR and no MMR protein deletion is non-dMMR; the interpretation method can adopt the standard of the College of American Pathologists (CAP) to determine whether the MMR protein expression is deleted; for example, PCR methods can also be used to detect microsatellite loci.

In some embodiments, PCR (polymerase chain reaction) and/or ICH (immunohistochemistry) assays are used to determine whether endometrial cancer is MSI-H or dMMR endometrial cancer, or non-MSI-H or non-dMMR endometrial cancer.

### Definitions and description

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

Herein, unless otherwise specified, the amount of anlotinib or a pharmaceutically acceptable salt thereof mentioned herein refers to the amount of the active ingredient anlotinib free base.

Unless otherwise specified, the term "dose" refers to a dose administered to a patient without considering the weight or the body surface area (BSA) of the patient. For example, a 60 kg human and a 100 kg human will receive the same dose of antibody (e.g., 240 mg of anti-PD-1 antibody).

The term "MSI" refers to microsatellite instability.

The term "MSI-H" refers to a microsatellite instability-high or microsatellite instability-high mutation (MSI-high).

The term "MSI-L" refers to microsatellite instability-low (MSI-low).

The term "MSS" refers to microsatellite stability.

The term "MMR" refers to mismatch repair.

The term "dMMR" refers to deficient mismatch repair.

The term "pMMR" refers to proficient mismatch repair.

As used herein, the term "therapeutic combination" refers to a combination of two or more active ingredients (administered as the respective active ingredients themselves, or as their respective derivatives like pharmaceutically acceptable salts or esters, prodrugs, or compositions) that are administered simultaneously or sequentially. The active substances can be administered to a subject simultaneously or sequentially in any order as a single formulation.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include an Fab fragment, an Fab' fragment, an F(ab')₂ fragment, an Fv fragment, an isolated CDR region, a single chain Fv molecule (scFv), an Fd fragment and other antibody fragments known in the art. The antibody and the fragment thereof may also include a recombinant polypeptide, a fusion protein, and a bispecific antibody. The anti-PD-L1 antibody and the fragment thereof described herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof described herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The term "humanized antibody" refers to an antibody comprising complementarity determining regions (CDRs) derived from a non-human antibody, and framework and constant regions derived from a human antibody. For example, an anti-PD-L1 antibody described herein may comprise CDRs derived from one or more murine antibodies as well as human framework and constant regions. Thus, in one embodiment, the humanized antibody described herein binds to the same epitope on PD-L1 as the murine antibody from which the CDRs of the humanized antibody are derived. In some embodiments, the anti-PD-L1 antibody is a humanized antibody. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies disclosed herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies described herein are reverted to the corresponding amino acids in the parent murine antibodies. For example, for the VH and VL of humanized 5G11 and humanized 13C5 antibodies, several sites of framework amino acids of the template human antibodies described above are reverted to the corresponding amino acid sequences in the mouse 5G11 and 13C5 antibodies. In one embodiment, the amino acids at positions 53, 60 and/or 67 of the light chain variable region are reverted to the corresponding amino acids found at the positions in mouse 5G11 or 13C5 light chain variable region. In another embodiment, the amino acids at positions 24, 28, 30, 49, 73, 83 and/or 94 of the heavy chain variable region are reverted to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative reverse mutations can be made in the framework regions of the humanized antibodies disclosed herein to improve the properties of the antibodies. The present application also includes humanized antibodies that bind to PD-L1 and comprise framework modifications corresponding to the exemplary modifications disclosed herein relative to any suitable framework sequence, as well as other framework modifications that otherwise improve antibody properties.

The present application describes an isolated antibody or a fragment thereof that binds to PD-L1, wherein the antibody can be produced by a hybridoma selected from the group consisting of the hybridomas designated herein as 13C5 and 5G11. The present application further provides isolated polynucleotides encoding the antibodies and the fragments thereof disclosed herein. The present application also includes expression vectors comprising the isolated polynucleotides, and host cells comprising the expression vectors.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-naturally occurring preparation of antibody molecules of an individual molecule component (i.e., antibody molecules whose base sequences are substantially identical and which exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof described herein is specific for PD-L1. In one embodiment, the antibody or the fragment thereof is specific for PD-L1. In one embodiment, the antibody or the fragment thereof described herein binds to human or primate PD-L1, but does not bind to PD- L1 from any other mammals. In another embodiment, the antibody or the fragment thereof does not bind to mouse PD-L1. The terms "human PD-L1", "hPD-L1" and "huPD-L1" and the like are used interchangeably herein and refer to human PD-L1 and variants or isotypes of human PD-L1. The terms "specific", "specificity" and "specifically" refer to that the antibody or fragment thereof binds to PD-L1 with greater affinity than any other targets.

The terms "treat", "treating" and "treatment" usually refer to acquiring needed pharmacological effect and/or physiological effect. In terms of partially or fully stabilizing or curing the disease and/or a side effect of the disease, the effect can be therapeutic. As used herein, "treat", "treating" and "treatment" encompass any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating a specific disease, condition or disorder; (ii) alleviating, ameliorating or eliminating one or more symptoms of a specific disease, condition or disorder, or (iii) preventing or delaying onset of one or more symptoms of the specific disease, condition or disorder described herein. The amount of active substance (e.g., the antibody or compound disclosed herein) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

The terms "administer", "administration" and "administering" refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include parenteral routes of administration (including but not limited to intravenous, intramuscular, subcutaneous, intraperitoneal, spinal, or other parenteral routes of administration), for example, by injection or infusion. In some emboidments, the phrase "parenteral administration" or "administered parenterally" as used herein are used interchangeably, and typically refer to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration. Other non-parenteral routes include local, epidermal or mucosal administration route, e.g., intranasal, intravaginal, intrarectal, sublingual or local administration route. Administration may also be performed, e.g., once, multiple times, and/or over one or more extended periods of time.

The term "pharmaceutically acceptable" is used herein for those compounds, materials, compositions, and/or dosage forms which are suitable for use in contact with the tissues of human beings and animals without excessive toxicity, irritation, allergic response, or other problems or complications.

The term "pharmaceutically acceptable salt" includes salts formed from a free base and an acid and salts formed from an acid and a free base, for example, hydrochloride, hydrobromide, nitrate, sulfate, phosphate, formate, acetate, trifluoroacetate, fumarate, oxalate, maleate, citrate, succinate, mesylate, benzenesulfonate and *p*-methylbenzenesulfonate; in some emboidments, the salts are hydrochloride, hydrobromide, sulfate, formate, acetate, trifluoroacetate, fumarate, maleate, mesylate, *p*-methylbenzenesulfonate, sodium salt, potassium salt, ammonium salt and amino acid salt, and the like. In the present application, in forming a pharmaceutically acceptable salt, the acid and the free base are in a molar ratio of 1:0.2-1:5; in some embodiment, the acid and the free base are in a molar ratio of 1:0.5, 1:1, 1:2, 1:3, 1:4, 1:5, 1:6, 1:7, or 1:8.

As used herein, the terms "subject" and "patient" are used interchangeably. In some embodiments, the term "subject" or "patient" refers to a mammal. In some embodiments, the subject or patient is a mouse. In some embodiments, the subject or patient is a human.

The term "unit dose" refers to the smallest unit of packaging containing a certain quantity of pharmaceutical product; for example, in a box of seven capsules, each capsule is a unit dose; or a vial of injection is a unit dose. The term "multiple dose" consists of multiple unit doses.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof disclosed herein to a subject.

Unless otherwise stated herein, singular terms encompass plural forms, and vice versa.

As used herein, unless otherwise stated, the terms "comprise", "comprises" and "comprising" or equivalents thereof are open-ended statements and mean that elements, components and steps that are not specified may be included in addition to those listed.

All patents, patent applications and other publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### DETAILED DESCRIPTION

For clarity, the present application is further described with the following examples, which are, however, not intended to limit the scope of the present application. All reagents are commercially available and can be used without further purification. In the examples, the anti-PD-L1 antibody was prepared as described in WO2016022630, and after affinity chromatography, an eluate containing the antibody was obtained by conventional antibody purification methods.

### Example 1 Phase II Clinical Trial for Endometrial Cancer

Endometrial cancer subjects meeting inclusion criteria were first tested for MMR/MSI status by immunohistochemical or PCR methods, and then grouped according to the MSI-H/dMMR test results; for non-MSI-H/non-dMMR subjects, an anti-PD-L1 antibody and anlotinib hydrochloride (cohort I) were administered, while for MSI-H or dMMR subjects, an anti-PD-L1 antibody (cohort II) was administered, and for patients with disease under control (CR + PR + SD) and tolerable adverse reactions, the administration could be continued until clinical benefits were lost, until the toxicity was intolerable, until PD/iCPD was given by efficacy evaluation, or until the investigator deemed further medication inappropriate. Efficacy evaluations were performed every 6 weeks (42 days) ± 3 days, starting on day 1 of treatment cycle 1, and the efficacy evaluations were performed once every 12 weeks (84 days) ± 3 days after 54 weeks, until the subjects developed disease progression confirmed by oncologic imaging.

### 1.1 Primary inclusion criteria:

1) Histologically or cytologically confirmed unresectable recurrent or metastatic advanced endometrial cancer (previously received 1-2 lines of systemic standard chemotherapy and failed or was intolerant; preliminary neoadjuvant or adjuvant chemotherapy was allowed).
2) It was confirmed that there was at least one measurable lesion according to the Response Evaluation Criteria in Solid Tumors (RECIST 1.1).
3) Age: ≥18 years; ECOG PS scoring: 0-1; an expected survival time of more than 3 months.

### 1.2 Test drug

Anti-PD-L1 antibody injection hu5G11-hIgG1: 1200 mg of the anti-PD-L1 antibody injection (strength: 600 mg/20 mL) was diluted to 250 mL with normal saline, and the infusion time was 60 ± 10 min (from the beginning of the infusion of the anti-PD-L1 antibody injection until the end of the infusion of the anti-PD-L1 antibody injection and the completion of flushing the tube with normal saline (20 mL recommended)). The anti-PD-L1 antibody injection was administered on the first day, and once every 21 days, that is, 21 days were counted as a treatment cycle (d1/q3w).

Anlotinib hydrochloride capsule (anlotinib dihydrochloride as active ingredient): once daily (orally taken before breakfast), 1 capsule (12 mg) each time. The oral administration was performed for 2 consecutive weeks and interrupted for 1 week, that is, 21 days were counted as a treatment cycle, and the drug was administered on days 1-14 of each cycle. Except in special circumstances, it was recommended to take it at a fixed time every day. (i.e., anlotinib hydrochloride capsule: 12 mg/qd, d1-14/q3w)

The investigator could adjust the dose of the anlotinib hydrochloride capsule, e.g., to 12 mg, 10 mg or 8 mg, according to the disease condition, safety and other aspects.

### 1.3 Evaluation criteria

Safety evaluation criteria: the severity of adverse events was determined using the NCI-CTC AE 5.0 criteria. Effectiveness evaluation criteria: disease status was determined using RECIST 1.1, iRECIST criteria. RECIST 1.1 criteria were used as the primary criteria, and iRECIST criteria were used to confirm efficacy. That is, subjects determined to be disease progression (PD) according to RECIST 1.1 criteria were further confirmed according to iRECIST criteria to determine whether to take further medication observation.

### 1.4 Endpoints

### Primary endpoint: objective response rate (ORR) for IRC evaluation

Secondary endpoint: (1) objective response rate (ORR), disease control rate (DCR), duration of response (DOR), progression-free survival (PFS), overall survival (OS), DOR rate (≥ 6 months), and the like evaluated by investigators; (2) the incidence and severity of adverse events (AEs) and serious adverse events (SAEs), and abnormal laboratory test indicators.

### 1.5 Results

Preliminary studies have shown that a combination of anlotinib hydrochloride and an anti-PD-L1 antibody can safely and effectively treat non-MSI-H and/or non-dMMR endometrial cancer with clinical benefit to the subject. Up to the day the data were acquired, in this study, 22 subjects in cohort I (the aritinib hydrochloride combined with anti-PD-L1 antibody group) completed at least 2 cycles of treatment, with 14 subjects (14/22) having the best efficacy of SD (stable disease), 5 subjects (5/22) having the best efficacy of PR (partial response), 1 subject (1/22) having the best efficacy of CR, objective response rate (ORR) up to 27.27%, disease control rate (DCR) up to 90.91%, adverse reaction incidence of 75%, and incidence of adverse reactions above grade 3 of 7%. The details are shown in Table 1.

Preliminary studies have shown that anti-PD-L1 antibody can safely and effectively treat MSI-H and/or dMMR tumors, particularly MSI-H and/or dMMR endometrial cancers, with clinical benefit to the subject. Up to the day the data were acquired, 6 subjects in cohort II (anti-PD-L1 antibody group) completed at least 2 cycles of treatment, with 2 subjects (2/6) having the best efficacy of SD (stable disease), 1 subject (1/6) having the best efficacy of PR (partial response), objective response rate up to 16.67%, disease control rate up to 50.00%, adverse reaction incidence of 67%, and incidence of adverse reactions above grade 3 of 16%; the details are shown in Table 1.

**Table 1. Summary of best response after completion of at least 2 cycles of treatment**

| Response | Number of subjects (ratio) | |
|---|---|---|
| | Cohort I | Cohort II |
| CR | 1/22 (4.54%) | 0/6 (0.00%) |
| PR | 5/22 (22.73%) | 1/6 (16.67%) |
| SD | 14/22 (63.64%) | 2/6 (33.33%) |
| PD | 2*/22 (9.10%) | 3**/6 (50.00%) |
| ORR | 6/22 (27.27%) | 1/6 (16.67%) |
| DCR | 20/22 (90.91%) | 3/6 (50.00%) |

| | | |
|---|---|---|
| * denotes: the current best efficacy in 1 of the subjects was iUPD (iRECIST criteria), and further evaluation of efficacy was required; ** denotes: the current best efficacy in 3 of the subjects was iUPD (iRECIST criteria), and further evaluation of efficacy was required. | | |

## Claims

1. Use of an anti-PD-L1 antibody or a pharmaceutical composition thereof in preparing a medicament for treating MSI-H and/or dMMR tumors, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

2. The use according to claim 1, wherein the MSI-H and/or dMMR tumor is selected from the group consisting of breast cancer, head and neck cancer, skin cancer, soft tissue sarcoma, respiratory system cancer, nervous system malignant tumor, digestive system tumor, endocrine system tumor, genitourinary system cancer, gynaecological cancer, and hematologic system malignant tumor.

3. The use according to claim 1 or 2, wherein the MSI-H and/or dMMR tumor is an MSI-H and/or dMMR malignant solid tumor.

4. The use according to any one of claims 1-3, wherein the MSI-H and/or dMMR tumor is MSI-H and/or dMMR endometrial cancer.

5. Use of a therapeutic combination comprising an anti-PD-L1 antibody, and anlotinib or a pharmaceutically acceptable salt thereof in preparing a medicament for treating endometrial cancer, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

6. The use according to claim 5, wherein the endometrial cancer is non-MSI-H endometrial cancer and/or non-dMMR endometrial cancer.

7. The use according to any one of claims 4-6, wherein the endometrial cancer is advanced endometrial cancer and/or refractory and/or recurrent and/or metastatic endometrial cancer.

8. The use according to any one of claims 1-7, wherein the anti-PD-L1 antibody or the pharmaceutical composition thereof is in a form for parenteral administration or in a form for intravenous administration.

9. The use according to any one of claims 1-8, wherein the anti-PD-L1 antibody is in the form of a pharmaceutical composition in which the anti-PD-L1 antibody has a concentration of 10-60 mg/mL.

10. The use according to any one of claims 1-9, wherein the anti-PD-L1 antibody or the pharmaceutical composition thereof is administered at a single dose of 600-2400 mg, or 600, 800, 1000, 1200, 1400, 1600, 1800, 2000, 2200 or 2400 mg.

11. The use according to any one of claims 1-10, wherein the anti-PD-L1 antibody or the pharmaceutical composition thereof is administered once a week, once every 2 weeks, once every 3 weeks, or once every 4 weeks.

12. The use according to any one of claims 5-6, wherein the anti-PD-L1 antibody and anlotinib or the pharmaceutically acceptable salt thereof may be administered simultaneously, nonsimultaneously, or sequentially.

13. The use according to any one of claims 5-6 and 12, wherein every 3 weeks are counted as one treatment cycle, the anti-PD-L1 antibody is administered on the first day of each cycle, and anlotinib or the pharmaceutically acceptable salt thereof is administered on days 1-14 of each cycle.

14. The use according to any one of claims 5-6 and 12-13, wherein the therapeutic combination is a formulation suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib or the pharmaceutically acceptable salt thereof.

15. The use according to claim 13 or 14, wherein 1200 mg of the PD-L1 antibody is administered on the first day of each treatment cycle, and 6 mg, 8 mg, 10 mg and/or 12 mg of anlotinib or the pharmaceutically acceptable salt thereof is administered daily on days 1-14 of each cycle.

16. A method for treating an MSI-H and/or dMMR tumor, comprising: administering to a patient in need thereof a therapeutically effective amount of an anti-PD-L1 antibody or a pharmaceutical composition thereof, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

17. The method according to claim 16, wherein the MSI-H and/or dMMR tumor is MSI-H and/or dMMR endometrial cancer.

18. A method for treating endometrial cancer, comprising: administering to a patient in need thereof a therapeutically effective amount of an anti-PD-L1 antibody, and anlotinib or a pharmaceutically acceptable salt thereof, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

19. The method according to claim 18, wherein the endometrial cancer is non-MSI-H endometrial cancer and/or non-dMMR endometrial cancer.

20. A therapeutic combination for use in treating endometrial cancer, comprising: an anti-PD-L1 antibody, and anlotinib or a pharmaceutically acceptable salt thereof. wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

21. The therapeutic combination according to claim 20, wherein the therapeutic combination comprises: a pharmaceutical composition comprising 600-2400 mg of the anti-PD-L1 antibody provided in multiple-dose form and a pharmaceutical composition comprising 6 mg, 8 mg, 10 mg and/or 12 mg of anlotinib or the pharmaceutically acceptable salt thereof in a unit dose.

22. The use according to any one of claims 1-15, or the method according to any one of claims 16-19, or the therapeutic combination according to any one of claims 20-21, wherein the anti-PD-L1 antibody comprises an amino acid sequence as follows: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

23. The use according to any one of claims 1-15, or the method according to any one of claims 16-19, or the therapeutic combination according to any one of claims 20-21, wherein the anti-PD-L1 antibody comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

24. The use according to any one of claims 1-15, or the method according to any one of claims 16-19, or the therapeutic combination according to any one of claims 20-21, wherein the anti-PD-L1 antibody comprises the following amino acid sequences: a heavy chain variable region having at least 80% homology to an amino acid sequence set forth in SEQ ID NO: 13 or SEQ ID NO: 14; and a light chain variable region having at least 80% homology to an amino acid sequence set forth in SEQ ID NO: 15 or SEQ ID NO: 16.

25. The use according to any one of claims 1-15, or the method according to any one of claims 16-19, or the therapeutic combination according to any one of claims 20-21, wherein the anti-PD-L1 antibody comprises: a heavy chain variable region selected from the group consisting of heavy chain variable regions of humanized antibodies hu13C5-hIgG1, hu13C5-hIgG4, hu5G11-hIgG1 and hu5G11-hIgG4; and a light chain variable region selected from the group consisting of light chain variable regions of humanized antibodies hu13C5-hIgG1, hu13C5-hIgG4, hu5G11-hIgG1 and hu5G11-hIgG4.
